# EUROPEAN PATENT APPLICATION

(11) **EP 1 955 991 A2**
(43) Date of publication of application: **13.08.2008**
(21) Application number: 08151133.9
(22) Date of filing: 06.02.2008
(51) Int. Cl.: C07C 22/00

(54) **Preparation of organic compounds bearing a trifluoromethyl group on a quaternary carbon**

(30) Priority: 06.02.2007 US 702742
(71) Applicant: Air Products and Chemicals, Inc., Allentown, PA 18195-1501 (US)
(72) Inventor: Lal, Gauri, Sankar, Whitehall, PA 18052 (US); Ulman, Michael, Mertztown, PA 19539 (US); Casteel, JR., William, Jack, Fountain Hill, PA 18015 (US)
(74) Representative: Muir, Benjamin M. J.

(57) **Abstract**

A method for preparing a molecule bearing a trifluoromethyl group on a quaternary carbon atom, includes providing a reactant having a quaternary carbon atom bearing a carboxylic acid group and an electron withdrawing group; and reacting the reactant with SF₄ in a solvent to substitute the carboxylic acid group with the trifluoromethyl group and provide a reaction product mixture including the molecule bearing the trifluoromethyl group on the quaternary carbon atom.

## Description

### BACKGROUND OF THE INVENTION

The present invention pertains to a method for preparing organic compounds bearing a trifluoromethyl group on a quaternary carbon atom, particularly such organic compounds useful as intermediates in the synthesis of pharmaceutical and liquid crystalline products.

Novel methodologies for the preparation of trifluoromethyl (CF₃) substituted organic compounds have attracted a great deal of attention. Compounds bearing this structural entity have found applications in diverse industrial products ranging from dyes and polymers to pharmaceutical and agrochemical products. See McClinton et al., "Trifluoromethylations and Related Reactions in Organic Chemistry." Tetrahedron 1992, 48, 6555. In medicinals and agrochemicals, the increased lipophilicity and hydrolytic stability that the CF₃-group imparts have profoundly influenced their biological activity. *Id.* Organic compounds bearing the trifluoromethyl group have also been used as components of liquid crystal displays. Pauluth et al., "Advanced Liquid Crystals for Television." J. Mater. Chem. 2004, 14, 1219.

While the preparation of compounds bearing a trifluoromethyl group on 1° or 2° carbons atoms can be carried out with relative ease, the introduction of the CF₃ -group at a quaternary carbon is still a synthetic challenge and only a few methods are available to effect this transformation. These involve the use of trifluoromethyl-containing building blocks in multi-step processes or relatively expensive trifluoromethylating agents. McBee et. al., "Diels-Alder Reactions with Fluorine-containing Olefins." J. Am. Chem. Soc. 1955, 77, 915, described a Diels-Alder reaction of 2-methyl-3,3,3-trifluoropropene with various dienes to prepare 1-trifluormethyl-1'-methyl cyclohexenes. A similar reaction was reported by Hanzawa et al., "Construction of Trifluoromethylated Quaternary Carbons via Diels-Alder Reactions of 2-(Trifluoromethyl)propenoic Acid Derivatives: Application to the Synthesis of 16,16,16-Trifluororetinal." J. Org. Chem. 1991, 56, 1718, using 2-carboalkoxy-1-trifluoropropene with dienes to prepare 1-trifluoromethyl-1'-carboethoxycyclohexenes. The Ruppert's reagent, trifluoromethyl trimethylsilane, has been used to introduce the trifluoromethyl group into organic compounds via its reaction with ketones as the first step in the preparation of compounds bearing the CF₃-group on a quaternary carbon atom. See Blazejewski et al., "Radical allylation of trifluoromethylated xanthates: use of DEAD for removing the allyltributyltin excess." Tett. Lett. 2001, 42, 859. Other methods for introducing the CF₃-group at a quaternary carbon atom of organic compounds include the reaction of alkylamines with CF₃NO₂ (Golitz et al., "A New Method for the Introduction of Trifluoromethyl Groups." Chem. Int. Ed. Eng. 1977, 16, 12, 854), the reaction of carbanions with S-(Trifluoromethyl)dibenzothiophenium salts (Umemoto et al., "New Method for Trifluoromethylation of Enolate Anions and Applications to Regio-, Diastereo- and Enantioselective Trifluoromethylation." J. Org. Chem. 1994, 59, 5692) and the reaction of trifluoromethyl iodide with enamines to generate α-trifluoromethyl ketones followed by alkylation at the carbon atom bearing the CF₃-group (Balko et al., "Total synthesis of (±)-8-trifluoromethyl abscisic acid." Tett. Lett. 1999, 40, 6347).

A more direct approach to the synthesis of compounds bearing a CF₃-group on a quaternary carbon involves the reaction of BrF₃ with dithiocarbonates at the α-position of a carboxylic acid ester group. See Rozen et al. "α-Trifluoromethylation of Secondary and Sterically Hindered Carboxylates with Use of BrF3." J. Org. Chem. 2004, 69, 7241. Dmowski et al., "Selective reactions of 1,1-cycloalkanedicarboxylic acids with SF4. A route to 1,1-bis(trifluoromethyl)cycloalkanes, 1-fluoroformyl-1-(trifluoromethyl)cycloalkanes and 1-(trifluoromethyl)-1-cycloalkanecarboxylic acids." J. Fl. Chem. 2000, 102, 141, reported on the reaction of 1,1-cyclohexane dicarboxylic acid with SF₄ for the preparation of 1-fluoroformyl-1-trifluoromethylcycloalkanes and 1-trifluoromethyl-1-cyclohexane carboxylic acid.

Despite the foregoing developments, it is desired to provide additional means for preparing organic compounds bearing a trifluoromethyl group on a quaternary carbon atom.

### BRIEF SUMMARY OF THE INVENTION

Accordingly, a first aspect of the invention comprises a method for preparing a molecule bearing a trifluoromethyl group on a quaternary carbon atom, said method comprising:
providing a reactant comprising a quaternary carbon atom bearing a carboxylic acid group and an electron withdrawing group, said electron withdrawing group preferably being other than a carboxylic acid group; and
reacting the reactant with SF₄ in a solvent to substitute the carboxylic acid group with the trifluoromethyl group and provide a reaction product mixture comprising the molecule bearing the trifluoromethyl group on the quaternary carbon atom.

A second aspect of the invention comprises a method for preparing a molecule bearing a trifluoromethyl group and an alkyl group on a quaternary carbon atom, said method comprising the sequential steps of:
providing a reactant comprising a quaternary carbon atom bearing a carboxylic acid group and an ester group;
reacting the reactant with SF₄ in a solvent to substitute the carboxylic acid group with the trifluoromethyl group; and substituting the alkyl group for the ester group to provide the molecule bearing the trifluoromethyl group and the alkyl group on the quaternary carbon atom.

A third aspect of the invention comprises a method for preparing a molecule bearing a trifluoromethyl group on a quaternary carbon atom, said method represented by the following equation: 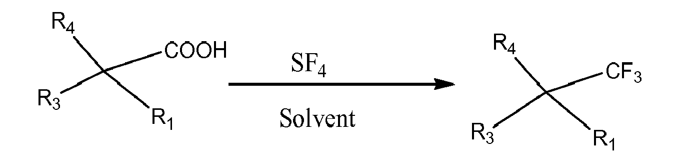 where:
(i) R₁ is an electron withdrawing group selected from: (a) carboxylic acid esters, COOR', where R' is unsubstituted alkyl, substituted alkyl, unsubstituted aryl, or substituted aryl of 1-20 carbon atoms; (b) NO₂; (c) SOR, where R is alkyl or aryl; (d) SO₂R, where R is alkyl or aryl; (e) POOR₃, where R is alkyl or aryl; (f) PR₃, where R is alkyl or aryl; and (g) CN; and
(ii) R₃,R₄ is cycloalkyl of 3-8 carbon atoms; or
(iii) R₃ and R₄ are independently selected from unsubstituted alkyl, substituted alkyl, unsubstituted aryl, and substituted aryl of 1-20 carbon atoms.

### DETAILED DESCRIPTION OF THE INVENTION

Herein, we describe a new procedure for the preparation of CF₃-substituted quaternary carbons. This method of the invention utilizes the reaction of SF₄ in a solvent with a carboxylic acid bearing an electron withdrawing group. In this process, the trifluoromethyl substituted product is obtained in good yields with a substituent group that can be further elaborated to produce a wide variety of products which are of potential utility as pharmaceuticals or liquid crystalline compounds.

The following equation summarizes an embodiment of the inventive method for obtaining compounds with a trifluoromethyl group on a quaternary carbon: 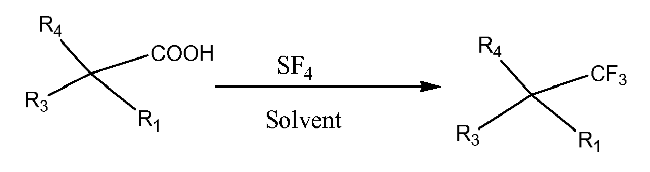 where R₁, R₃ and R₄ are defined as follows.

R₁ is preferably an electron withdrawing group and more preferably a carboxylic acid ester, COOR', where R' is alkyl or aryl of 1-20 carbon atoms with or without substituents, including heteroatoms such as O, N or S. In less preferred embodiments, R₁ is a group that does not withdraw electrons. Much lower yields of products are obtained where R₁ is not an electron withdrawing group. For example, when R₃,R₄ is 4-(4'propylcyclohexyl)cyclohexane and R₁ is not an electron withdrawing group, the yield of the CF₃ containing product is 5% versus 50% when R₁ is COOCH₃.

Additional non-limiting examples of suitable electron withdrawing groups include NO₂, SOR (where R is alkyl or aryl), SO₂R (where R is alkyl or aryl), POOR₃ (where R is alkyl or aryl), PR₃ (where R is alkyl or aryl) and CN.

In certain embodiments, R₃,R₄ is cycloalkyl of 3-8 carbon atoms with or without alkyl, aryl, cycloaliphatic and/or heteroatom (such as but not limited to O, S, N, and halogens such as Cl, F, Br, I ) substituents. The alkyl, aryl and cycloaliphatic substituents may be further substituted with substituents such as but not limited to alkyl, aryl, cycloalkyl (3-8 carbons) and alkylcycloalkyl, and all said substituents may have heteroatom substituents such as but not limited to O, S, N, and halogens such as Cl, F, B r, I.

In certain embodiments, R₃ is alkyl (preferably up to 20 carbon atoms), aryl (preferably up to 20 carbon atoms) with or without heteroatom substituents such as but not limited to O, S, N, and halogens such as Cl, F, Br, I.

In certain embodiments, R₄ is alkyl (preferably up to 20 carbon atoms), or aryl (preferably up to 20 carbon atoms) with or without heteroatom substituents such as but not limited to O, S, N, and halogens such as Cl, F, Br, I.

In certain embodiments, the reactant reacted with SF₄ is represented by a formula selected from the following group: 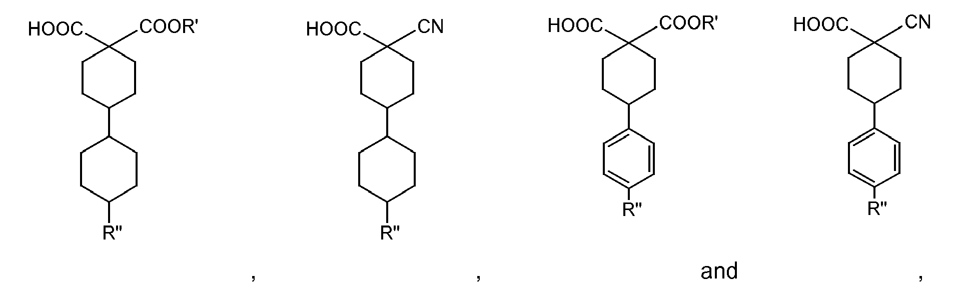 where R' is C₁-C₁₀ alkyl and R" is C₁-C₅ alkyl.

The solvent preferably comprises HF. Other suitable solvents include but are not limited to ethers, such as diethyl ether, THF (tetrahydrofuran), hydrocarbons, such as hexane, and combinations thereof. Other suitable solvents include but are not limited to fluorocarbons, such as, e.g., Freon 113. It is preferred that the solvent not react with the SF₄.

The preferred temperature range for the reaction is from -78°C to 100°C.

In certain embodiments, the reaction time ranges from 1 min to 5 days, or is about 24 hours.

The final product may optionally be purified prior to further use by, e.g., standard purification procedures, such as recrystallization, distillation or chromatography.

Optionally, the electron withdrawing substituents on the carbon bearing the CF₃-group can be converted to other groups by virtue of their chemical reactivity. Non-limiting examples of the conversion of an ester functionality to another group are illustrated below in Schemes 1-3.

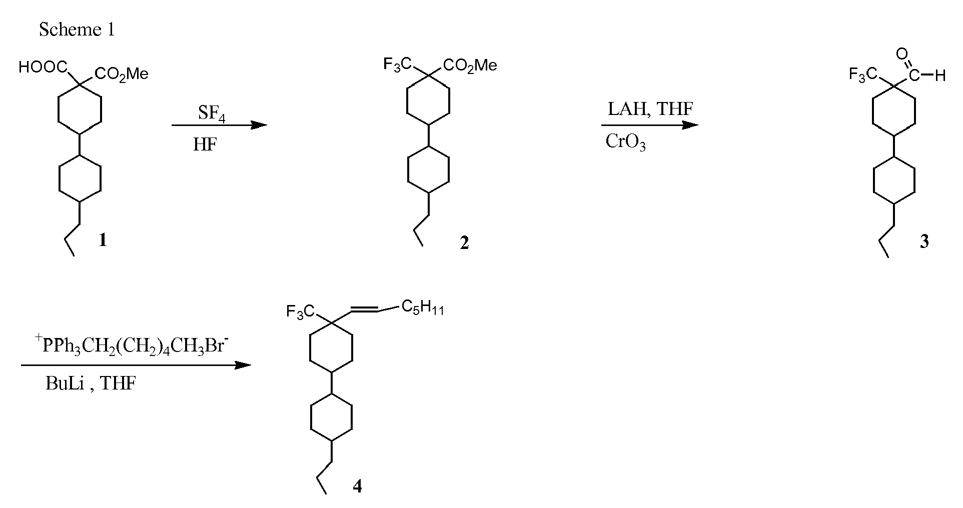

In Scheme 1, the carboxylic acid **1** is converted to the trifluoromethyl derivative **2.** The ester **2** is then transformed to the aldehyde **3** by reduction with lithium aluminum hydride and oxidation of the 1° alcohol formed. A Wittig reaction on the aldehyde affords the vinyl substituted product,**4.**

An alternative method to convert the ester group to another group is shown in Scheme 2.

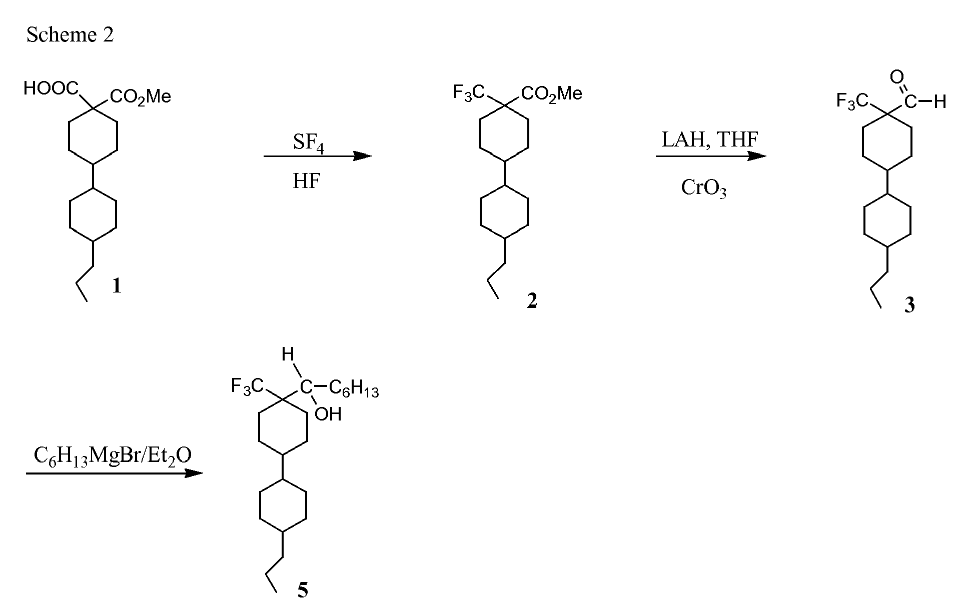

The carboxylic acid **1** is converted to the trifluoromethyl derivative **2.** The ester of **2** is then transformed to the aldehyde by reduction with lithium aluminum hydride and oxidation of the 1° alcohol formed. Reaction of the aldehyde **3** with hexyl magnesium bromide results in formation of alcohol **6.**

Thus, in Schemes 1 and 2, the invention comprises the steps of: (1) providing a reactant comprising a carboxylate group geminal to an ester group; (2) substituting a trifluoromethyl group for the carboxylate group; (3) converting the ester to an aldehyde; and (4) converting the aldehyde to a vinyl-substituted or alcohol-substituted intermediate.

In Scheme 3, the ester functionality is converted to a ketone group. Hydrolysis of the ester to the acid **7,** followed by conversion to the corresponding acid chloride, **8** and subsequent reaction with an organomagnesium or organocuprate reagent will generate the ketone **9.**

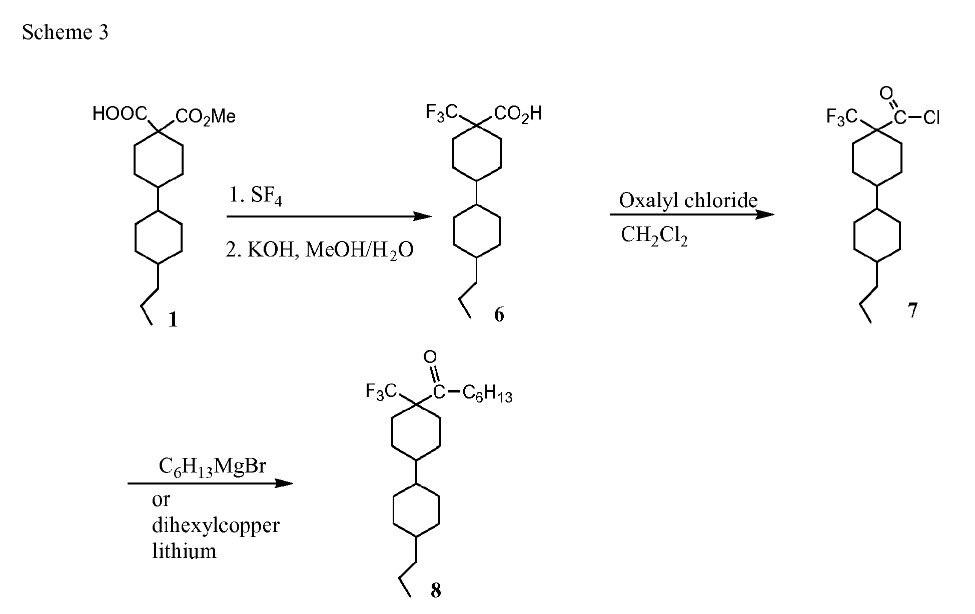

Thus, in Scheme 3, the invention comprises the steps of: (1) providing a reactant comprising a carboxylate group geminal to an ester group; (2) substituting a trifluoromethyl group for the carboxylate group; (3) converting the ester to an acid; (4) converting the acid to an acid chloride; (5) reacting the acid chloride with an organometallic reagent to form a ketone.

### EXAMPLES

The invention will be illustrated in more detail with reference to the following Examples, but it should be understood that the present invention is not deemed to be limited thereto.

### Example 1:

### Fluorination of 1-carboxy-1-carbomethoxy-4(4'-propylcyclohexyl)cyclohexane with sulfur tetrafluoride

4.2 g of 1-carboxy-1-carbomethoxy-4(4'-propylcyclohexyl)cyclohexane were placed in a 75 mL Hoke cylinder fitted with a tee connected to a relief device and an inlet valve and containing a magnetic stir bar. The Hoke cylinder was evacuated and cooled to -78°C. 9.4 g of hydrogen fluoride were vacuum transferred into the cooled cylinder along with 9.5 molar equivalents of sulfur tetrafluoride. The valve on the cylinder was closed and it was allowed to warm up to room temperature. The reaction was allowed to stir at room temperature for 24 hours. The volatiles were evacuated from the Hoke cylinder and the residual contents were extracted with diethyl ether and neutralized over sodium bicarbonate. The mixture was filtered, concentrated and passed through a plug of silica gel, eluting with 2-4% ethyl acetate in hexanes (vol/vol). A GC-MS chromatogram showed four product peaks, corresponding to the four axial/equatorial isomers, each with a molecular ion peak (m/z = 334). Upon solvent evaporation, the reaction yielded 2.0 g of product.

### Example 2:

### Reduction of 1-trifluoromethyl-1-carbomethoxy-4-(4'-propylcyclohexyl)cyclohexane with lithium aluminum hydride

200 milligrams of lithium aluminum hydride were placed in a dry two-neck 100 mL round bottom flask under nitrogen. 10 mL anhydrous tetrahydrofuran were added and the flask was cooled to 0°C in an ice bath. 1.45 g of 1-trifluoromethyl-1-carbomethoxy-4-(4'-propylcyclohexyl)cyclohexane were dissolved in 6 mL anhydrous tetrahydrofuran and added dropwise to the reaction flask. The reaction was stirred for 1 hour at 0°C and then 4 hours at room temperature. The reaction mixture was diluted with diethyl ether and 0.25 mL water was slowly added, followed by 0.25 mL 15% NaOH(aq), followed by 0.75 mL water. The mixture was stirred overnight. After filtration of the aluminum salts, the organic phase was washed twice with water, dried over magnesium sulfate, filtered and concentrated to yield 1.0 g of product. A GC-MS chromatogram showed three distinct peaks (presumably the fourth was hidden under one of the other three) for the axial/equatorial isomers, each having a molecular ion peak (m/z = 306).

### Example 3:

### Oxidation of 1-trifluoromethyl-1-hydroxymethyl-4-(4'-propylcyclohexyl)cyclohexane with chromic anhydride/pyridine

1.0 g of chromic anhydride was placed in a dry two-neck round bottom flask under nitrogen. 10 mL anhydrous methylene chloride were added followed by 1.7 mL anhydrous pyridine. The mixture was allowed to stir for 40 minutes at room temperature. 1.0 g of 1-trifluoromethyl-1-hydroxymethyl-4-(4'-propylcyclohexyl)cyclohexane was added in 6 mL anhydrous methylene chloride and allowed to stir over a weekend. The reaction was diluted with ether and the precipitate was filtered through celite. The organic phase was washed twice with dilute HCl solution, once with NaHCO₃ solution and once with water, followed by drying over magnesium sulfate and evaporation of the solvents to yield 0.59 g of material. A GC-MS chromatogram showed peaks with a molecular ion peak (m/z = 302).

### Example 4:

### Wittig reaction of the 1-trifluoromethyl-1-formyl-4-(4'-propylcyclohexyl)cyclohexane

6.25 grams of hexyl phosphonium bromide were placed in a two-neck dry round bottom flask under nitrogen. 30 mL anhydrous tetrahydrofuran were added and cooled to -70°C. 5.8 mL of 2.5M n-butyl lithium in hexanes were added dropwise with stirring and all the salts dissolved to form an orange solution. The cooling bath was lowered slightly and 4.04 g of 1-trifluoromethyl-1-formyl-4-(4'-propylcyclohexyl)cyclohexane in 10 mL tetrahydrofuran were added slowly and allowed to stir two hours. Diethyl ether was added and the reaction mixture was washed with dilute HCl (aq) and subsequently with saturated sodium bicarbonate solution. The organic phase was concentrated and triturated with hexanes. The hexanes were passed through a plug of silica, which was subsequently washed with additional hexanes. The combined hexanes solvent was evaporated to yield 4.21 g of product. A GC-MS chromatogram showed a molecular ion peak (m/z= 372).

## Claims

1. A method for preparing a molecule bearing a trifluoromethyl group on a quaternary carbon atom, said method comprising:
reacting a reactant, comprising a quaternary carbon atom bearing a carboxylic acid group and an electron withdrawing group other than a carboxylic acid, with SF₄ in a solvent to substitute the carboxylic acid group with the trifluoromethyl group and provide a reaction product mixture comprising the molecule bearing the trifluoromethyl group on the quaternary carbon atom.

2. The method of claim 1, said method represented by the following equation: 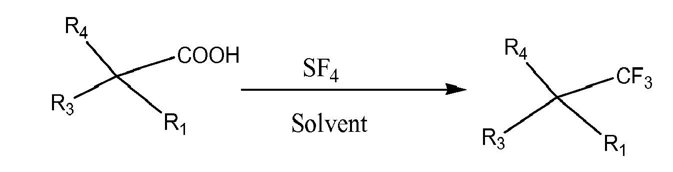 where:
(i) R₁ is an electron withdrawing group selected from: (a) carboxylic acid esters, COOR', where R' is unsubstituted alkyl, substituted alkyl, unsubstituted aryl, or substituted aryl of 1-20 carbon atoms; (b) NO₂; (c) SOR, where R is unsubstituted or substituted alkyl or aryl; (d) SO₂R, where R is unsubstituted or substituted alkyl or aryl; (e) POOR₃, where R is unsubstituted or substituted alkyl or aryl; (f) PR₃, where R is unsubstituted or substituted alkyl or aryl; and (g) CN; and either
(ii) R₃,R₄ is unsubstituted or substituted cycloalkyl of 3-8 carbon atoms; or
(iii) R₃ and R₄ are independently selected from unsubstituted alkyl, substituted alkyl, unsubstituted aryl, and substituted aryl of 1-20 carbon atoms.

3. The method of any preceding claim, wherein the reactant is represented by a formula selected from: 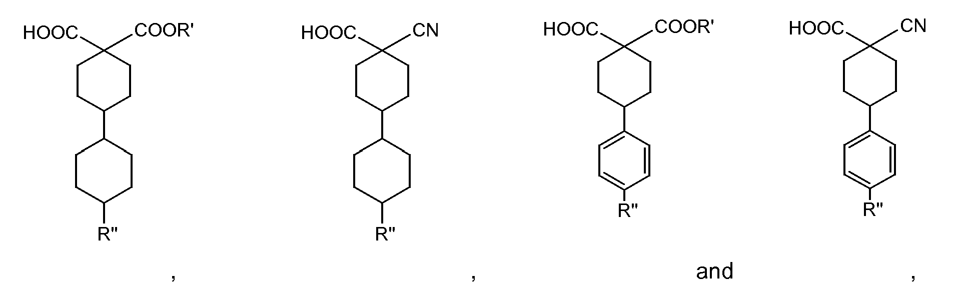 where R' is C₁-C₁₀ alkyl and R" is C₁-C₅ alkyl.

4. The method of any preceding claim, wherein the electron withdrawing group is an ester.

5. The method of any preceding claim, wherein subsequent to the reacting step, the electron withdrawing group is converted to another group selected from aldehyde, alcohol, acid chloride, ketone, and olefin.

6. The method of claim 5, wherein the electron withdrawing group is an ester and the ester is converted to an aldehyde which is further converted to an alcohol.

7. The method of claim 5, wherein the electron withdrawing group is an ester and the ester is converted to an acid which is converted to to an acid chloride, the acid chloride is converted to a ketone.

8. The method of Claim 4, the method further comprising, subsequent to the reacting step, substituting an alkyl group for the ester group to provide a molecule bearing the trifluoromethyl group and the alkyl group on the quaternary carbon atom.

9. The method of claim 8, wherein the substituting step comprises converting the ester to an aldehyde, converting the aldehyde to a vinyl-substituted or alcohol-substituted intermediate, and hydrogenating the intermediate.

10. The method of claim 8, wherein the substituting step comprises converting the ester to an acid, converting the acid to an acid chloride, reacting the acid chloride with an organometallic reagent to form a ketone, and reducing the carbonyl group of the ketone to a methylene group.

11. The method of any preceding claim, wherein the solvent comprises at least one member selected from HF, ethers, THF, hydrocarbons and fluorocarbons.

12. The method of claim 11, wherein the solvent comprises HF.

13. The method of any preceding claim, further comprising purifying the molecule from the reaction product mixture by at least one of recrystallization, distillation and chromatography.
